# EUROPÄISCHE PATENTANMELDUNG

(11) **EP 1 113 259 A2**
(43) Veröffentlichungstag der Anmeldung: **04.07.2001**
(21) Anmeldenummer: 00124966.3
(22) Anmeldetag: 16.11.2000
(51) Int. Cl.: G01N 11/04, G01N 33/32

(54) **Verfahren und Vorrichtung zur Feststellung von Veränderungen flüssiger Medien, insbesondere Überzugsmittel bei Scherbelastung**

(30) Priorität: 04.12.1999 DE 19958489
(71) Anmelder: E.I. DU PONT DE NEMOURS AND COMPANY, Wilmington Delaware 19898 (US)
(72) Erfinder: Rupieper, Paul, 42111 Wuppertal (DE); Höffer, Michael, 42349 Wuppertal (DE); Voyé, Christian, 58285 Gevelsberg (DE)
(74) Vertreter: Gille Hrabal Struck Neidlein Prop Roos

(57) **Zusammenfassung**

Verfahren und Vorrichtung zur Feststellung von durch Scherbelastung bedingten Veränderungen eines flüssigen Mediums (6), insbesondere eines flüssigen Überzugsmittels oder dessen Komponenten, bei dem man ein gegebenes Volumen des Mediums wiederholt ein Scherelement (3) unter reproduzierbaren Bedingungen passieren läßt.

## Beschreibung

Die vorliegende Erfindung betrifft ein Verfahren zur Untersuchung und Feststellung von Veränderungen an flüssigen Medien, insbesondere Überzugsmitteln und deren Komponenten durch Scherbelastung sowie eine Vorrichtung zur Durchführung des Verfahrens.

Disperse Bestandteile enthaltende flüssige Medien können irreversible Veränderungen oder Schädigungen erfahren, wenn sie einer Scherbelastung unterliegen.

Bei der Serienlackierung zwecks Lackversorgung der Sprühorgane in Ringleitungssystemen geführte Flüssiglacke können typische Beispiele für einer Scherbelastung unterliegende, disperse Bestandteile enthaltende, flüssige Medien sein. Die Flüssiglacke werden dabei aus einem Vorratsgefäß in die Ringleitung gepumpt, eine Teilmenge über die Sprühorgane während der Lackapplikation entnommen und der nicht entnommene Rest wieder in das Vorratsgefäß zurückgefördert und dabei mit dem darin befindlichen Lackmaterial vermischt. Während der Flüssiglack im Vorratsgefäß drucklos ist, steht er in der Ringleitung unter Druck von beispielsweise bis zu 12 bar. Der im Kreislauf geführte Flüssiglack erfährt innerhalb der Ringleitung Druckdifferenzen, die sich beispielsweise in Fließrichtung betrachtet vor und hinter innerhalb der Ringleitung installierten Bauteilen ergeben, insbesondere beispielsweise vor und hinter Pumpen und Ventilen. Die dabei auf den Flüssiglack einwirkende Scherbelastung kann zur Schädigung disperser Bindemittelbestandteile und/oder von Pigmenten führen. Das Ausmaß der Schädigung kann soweit gehen, daß der Flüssiglack nicht mehr verwendbar ist. Die Schädigung kann beispielsweise in einer unerwünschten und irreversiblen Veränderung des rheologischen Verhaltens, einer Stippenbildung im Lack und/oder in Effekt- oder Farbtonveränderungen bestehen. Die Scherstabilität eines gegebenen Flüssiglackes ist nicht exakt bekannt. Die Prüfung auf derartige Veränderungen erfolgt in der Praxis empirisch, beispielsweise durch Probenahme und übliche anwendungstechnische Prüfungen. Die Flüssiglackproben haben jeweils keiner definierten, reproduzierbaren Scherbelastung unterlegen.

Zur Prüfung der Scherstabilität oder Ringleitungsstabilität von Flüssiglacken ist es bekannt, eine Menge des zu prüfenden Flüssiglackes magnetisch zu rühren und jeweils durch Probenahme nach einem definierten Zeitraum auf Veränderungen des Lackmaterials zu untersuchen. Dieses Verfahren korreliert nur bedingt mit den Scherbelastungen in einer Ringleitungsinstallation und leidet insbesondere an einer mangelnden Reproduzierbarkeit.

Aufgabe der Erfindung ist die Bereitstellung eines reproduzierbaren Verfahrens zur Untersuchung der Auswirkung von Scherbelastungen auf flüssige Medien, insbesondere flüssige Überzugsmittel.

Die Lösung der Aufgabe gelingt, indem man ein gegebenes Volumen eines flüssigen Mediums wiederholt einer Scherung unter jeweils definierten Bedingungen unterwirft.

Gegenstand der Erfindung ist daher ein Verfahren zur Feststellung von reversiblen oder irreversiblen Veränderungen eines flüssigen Mediums bei Ausübung einer Scherbelastung, das dadurch gekennzeichnet ist, daß man ein gegebenes Volumen des zu untersuchenden flüssigen Mediums wiederholt ein Scherelement unter reproduzierbaren Bedingungen passieren läßt.

Im erfindungsgemäßen Verfahren läßt man ein gegebenes Volumen, beispielsweise 500 bis 2000 ml eines einer Scherbelastung auszusetzenden flüssigen Mediums wiederholt ein Scherelement jeweils unter definierten Bedingungen passieren. Im allgemeinen wird die Scherbelastung bei Temperaturen von beispielsweise 20 bis 50°C durchgeführt. Es ist zweckmäßig, daß man das gegebene Volumen des flüssigen Mediums bei jeder Scherelementpassage jeweils möglichst vollständig das Scherelement passieren läßt. Bevorzugt liegt der Anteil des pro Scherelementpassage nicht gescherten Materials bei unter 5 %, besonders bevorzugt unter 1 %.

Das flüssige Medium passiert wiederholt das Scherelement, d.h. eine vorgegebene Anzahl von beispielsweise 50 bis 2000 Passagen, und durchläuft bei Passieren des Scherelementes ein Schergefälle von beispielsweise zwischen 100 und 10⁷ s⁻¹. Die Scherbelastung des flüssigen Mediums ergibt sich dabei aus dem herrschenden Schergefälle und der Anzahl der wiederholten Passagen des Scherelementes. Das Schergefälle selbst ergibt sich bei gegebenem flüssigen Medium aus der in Fließrichtung betrachtet vor und hinter dem Scherelement herrschenden Druckdifferenz P2-P1. Direkt vor dem Scherelement staut sich das flüssige Medium unter dem Eingangsdruck Pl von beispielsweise 5 bis 50 bar und die Fließgeschwindigkeit ist gering, bei Passieren des Scherelementes ist die Fließgeschwindigkeit hoch, bei Verlassen des Scherelementes entspannt sich das flüssige Medium auf den Druck P2 < P1 und die Fließgeschwindigkeit ist wieder gering. P2 entspricht im allgemeinen dem umgebenden Atmosphärendruck oder geringfügig darüber. Bei gegebenem flüssigen Medium, gegebener Temperatur und gegebenem Druck P2 des flüssigen Mediums hängt die Höhe des Schergefälles ab vom Eingangsdruck P1 vor dem Scherelement und vom Querschnitt des Scherelementes. Dem Fachmann ist es klar, daß im Falle nicht ideal kreisförmiger Querschnitte im Scherelement ein zusätzlicher Einfluß auf P1 und damit auf das Schergefälle besteht.

Als Scherelement dient eine Querschnittsverengung innerhalb eines geschlossenen, das gegebene Volumen des flüssigen Mediums enthaltenden, entlüfteten Leitungssystems. Der entlüftete Zustand garantiert, daß das flüssige Medium als inkompressible oder praktisch inkompressible Flüssigkeit vorliegt. Das Leitungssystem als solches besitzt einen Querschnitt, der keine nennenswerte Scherung des flüssigen Mediums bewirkt. Die als Scherelement dienende Querschnittsverengung ist hingegen so bemessen, daß das flüssige Medium bei Passieren des Scherelementes geschert wird. Beispielsweise liegt der Querschnitt im Leitungssystem oberhalb 10 mm², beispielsweise bei 10 bis 10000 mm², während der Querschnitt im Scherelement beispielsweise von 0,5 bis 10 mm², bevorzugt unter 5 mm² beträgt. Die Querschnittsverengung kann einen festen Wert besitzen, bevorzugt handelt es sich jedoch um eine einstellbare Querschnittsverengung, beispielsweise so daß das Schergefälle bei unter einem gegebenen Eingangsdruck P1 stehenden flüssigen Medium auf einen gewünschten Wert eingestellt werden kann. Bei dem Scherelement kann es sich beispielsweise zweckmäßig um einen mit einer Mikrometerschraube beispielsweise auf eine Spaltbreite von 0,1 bis 3 mm einstellbaren Spalt handeln.

Das Leitungssystem kann als geschlossener Kreislauf für das flüssige Medium ausgebildet sein. Ein einzelner darin vollzogener vollständiger Kreislauf des gesamten Volumens des flüssigen Mediums entspricht dann einer einzelnen Scherelementpassage. Bevorzugt handelt es sich jedoch um ein Leitungssystem, in dem das gegebene Volumen des flüssigen Mediums im Pendelverfahren unter wiederholtem Wechsel der Fließrichtung jeweils möglichst vollständig das Scherelement passieren kann.

Im erfindungsgemäßen Verfahren werden flüssige Medien einer definierten und reproduzierbaren Scherbelastung unterworfen, insbesondere um deren Scherstabilität untersuchen zu können. Beispielsweise kann untersucht werden, ob eine Veränderung oder Schädigung eines flüssigen Mediums unter Scherbelastung eintritt, ob eine unter Scherbelastung eingetretene Veränderung oder Schädigung reversibel oder irreversibel ist, wie schnell, d.h. nach wieviel Passagen des Scherelementes eine Veränderung oder Schädigung des flüssigen Mediums eintritt oder wie groß das Ausmaß der Veränderung oder Schädigung des flüssigen Mediums nach einer vorgegebenen Scherbelastung, d.h. einer vorgegebenen Anzahl von Passagen des Scherelementes ist. Das erfindungsgemäße Verfahren ermöglicht eine sachgerechte Beantwortung dieser Fragen. Schließlich wird bei jeder einzelnen Passage des Scherelementes das gesamte oder zumindest annähernd das gesamte Volumen des flüssigen Mediums gleichmäßig geschert, die Scherbelastung als solche erfolgt somit definiert und reproduzierbar.

Zur Gewährleistung einer definierten und reproduzierbaren Scherbelastung kann das Schergefälle während eines aus einer definierten Anzahl von Scherelementpassagen bestehenden Scherzyklus konstant gehalten werden, was auch bevorzugt ist. Dazu kann es beispielsweise notwendig sein, eine mögliche, sich als Folge einer Veränderung des flüssigen Mediums während der Scherbelastung ergebende Temperaturänderung oder Änderung des Eingangsdrucks P1 zu kompensieren. Beispielsweise kann es notwendig sein das flüssige Medium zu thermostatisieren oder den Eingangsdruck P1 an ein sich infolge Scherbelastung verändertes rheologisches Verhalten des flüssigen Mediums anzupassen und damit das Schergefälle konstant zu halten. Der Eingangsdruck P1 kann durch Veränderung der Querschnittsverengung im Scherelement und/oder durch Veränderung der in Fließrichtung betrachtet vor dem Scherelement auf das flüssige Medium einwirkenden Kraft geregelt werden.

In einer zweiten Ausführungsform des erfindungsgemäßen Verfahrens kann mit einem sich während eines Scherzyklus infolge Materialveränderung verändernden Schergefälle gearbeitet werden, indem keine der vorstehend erläuterten Kompensationsmaßnahmen ergriffen wird. Auch hier ist eine definierte und reproduzierbare Scherbelastung des flüssigen Mediums gewährleistet.

In einer dritten Ausführungsform des erfindungsgemäßen Verfahrens wird innerhalb eines Scherzyklus nur ein Parameter unter Konstanthaltung der weiteren Parameter gezielt variiert. Beispiele für variable Parameter sind Eingangsdruck P1, Querschnitt des Scherelementes und Temperatur des flüssigen Mediums.

Bei den im erfindungsgemäßen Verfahren einer Scherbelastung auszusetzenden flüssigen Medien handelt es sich beispielsweise um Mehrphasensysteme im Sinne von disperse, flüssige oder feste Bestandteile enthaltenden flüssigen Medien. Insbesondere handelt es sich um disperse Bestandteile wie Pigmente, Füllstoffe und/oder disperse Bindemittel enthaltende flüssige Überzugsmittel. Es kann sich um pigmenthaltige oder pigmentfreie flüssige Überzugsmittel handeln. Die flüssigen Überzugsmittel können lösemittelfrei, lösemittelhaltig oder wäßrig sein. Es kann sich um Einkomponentenlacke oder auch um einzelne Komponenten von Mehrkomponentenlacken handeln. Es kann sich um physikalisch trocknende oder chemisch vernetzende flüssige Überzugsmittel handeln. Im Zusammenhang mit der vorliegenden Erfindung schließt der Ausdruck "Überzugsmittel" auch zur Herstellung flüssiger Überzugsmittel geeignete flüssige Bindemittel und Halbfabrikate ein. Beispiele sind dispers vorliegende oder disperse Bestandteile enthaltende Bindemittel, beispielsweise wäßrige oder nichtwäßrige Bindemitteldispersionen oder -emulsionen. Beispiele für flüssige Überzugsmittel im engeren Sinne sind flüssige Klarlacke, farb-und/oder effektgebende Basislacke, Decklacke, Pigmentpasten und Füllerlacke.

In allen Ausführungsformen betrifft die vorliegende Erfindung auch ein Verfahren zur Feststellung von reversiblen oder irreversiblen Veränderungen eines flüssigen Mediums bei Scherbelastung, das dadurch gekennzeichnet ist, daß man ein gegebenes Volumen des flüssigen Mediums wiederholt ein Scherelement unter definierten Bedingungen passieren läßt, wobei das flüssige Medium während und/oder nach Abschluß des aus wiederholten Scherelementpassagen bestehenden Scherzyklus auf Materialveränderung hin untersucht wird.

Während und/oder nach Beendigung eines Scherzyklus kann das scherbelastete flüssige Medium auf infolge Scherbelastung eingetretene reversible oder irreversible Veränderungen oder Schädigungen untersucht werden. Dazu kann ein- oder mehrmals innerhalb eines aus einer Anzahl von Passagen bestehenden Scherzyklus eine Probe des flüssigen Mediums, beispielsweise 10 bis 300 ml entnommen werden oder es wird die zur Verfügung stehende Gesamtmenge des flüssigen Mediums zur Untersuchung herangezogen. Eine Probenentnahme kann jederzeit erfolgen, vorzugsweise jedoch jeweils nur nach einer einzelnen abgeschlossenen Scherelementpassage des im Leitungssystem befindlichen flüssigen Mediums. Es ist vorteilhaft, daß eine Probenentnahme während des Scherzyklus keinerlei Einfluß auf die definierte Scherbelastung des flüssigen Mediums und die Reproduzierbarkeit des erfindungsgemäßen Verfahrens hat.

Bei Untersuchungen an entnommenen Proben kann es sich um direkt am flüssigen Medium durchführbare Untersuchungen oder Messungen handeln oder es handelt sich um indirekte Untersuchungen des flüssigen Mediums, beispielsweise anwendungstechnische Prüfungen auf Funktion bzw. Verwendbarkeit. Beispiele für direkt am flüssigen Medium, insbesondere flüssigen Überzugsmittel durchführbare Untersuchungen oder Messungen sind Viskositätsmessungen, Messung der Resonanzfrequenz des flüssigen Materials mittels Viskositätsmeßgeräten, visuelle Beurteilung, Prüfung auf Stippenbildung, Prüfung des Absetzverhaltens, optische Messungen am Naßlack, beispielsweise Naßlackfarbmetrik. Beispiele für indirekte Untersuchungen der flüssigen Medien, insbesondere der flüssigen Überzugsmittel sind Prüfung der Verarbeitbarkeit sowie Prüfungen an aus den flüssigen Überzugsmitteln aufgebrachten und gegebenenfalls getrockneten oder gehärteten Überzugsschichten, beispielsweise Bestimmung der Ablaufgrenze, mikroskopische Untersuchungen an Aufzügen, Farbmetrik, Helligkeits- und Glanzmessungen, visuelle Beurteilung, Erstellung von Eigenschafts/Schichtdicke-Korrelationsdiagrammen gemäß der in EP-B 0 842 414 beschriebenen Verfahrensweise.

Die Untersuchung des scherbelasteten flüssigen Mediums kann gegebenenfalls auch am innerhalb des Leitungssystems befindlichen flüssigen Medium durchgeführt werden, entweder bei stationärem oder bei strömendem flüssigen Medium. Bei Untersuchungen am innerhalb des Leitungssystems befindlichen flüssigen Medium handelt es sich um direkt am flüssigen Medium durchgeführte Messungen, beispielsweise inline-Temperaturmessung vor und/oder hinter dem Scherelement, inline-Messung des Eingangsdrucks P1 und/oder des Drucks P2, insbesondere auch die inline-Messung rheologischer Daten oder der Resonanzfrequenz des flüssigen Mediums mittels einer Meßeinheit mit Schwingungsgeber (Resonator). Die durch inline-Messungen erhaltenen Meßwerte können insbesondere auch zur Steuerung der bei der Erläuterung der ersten Ausführungsform des erfindungsgemäßen Verfahrens zur Ausübung einer Scherbelastung erwähnten Kompensationsmaßnahmen verwendet werden.

Auch die Messung der zur Erzeugung des Drucks P1 auf das flüssige Medium einwirkenden notwendigen Kraft oder die Bestimmung der Strömungsgeschwindigkeit des flüssigen Mediums kann herangezogen werden zur indirekten Bestimmung des Zustandes oder einer Veränderung des scherbelasteten flüssigen Mediums. Die Strömungsgeschwindigkeit des flüssigen Mediums kann beispielsweise indirekt bestimmt werden durch Weg/Zeitmessung am Dosiersystem bei bekanntem Querschnitt des Dosiersystems.

Insbesondere als Meßreihen anfallende Meßwerte können beispielsweise mittels Rechner (PC) gespeichert und ausgewertet werden.

Die inline oder an entnommenen Proben des flüssigen Mediums durchgeführten Untersuchungen dienen zur Bestimmung des Zustandes respektive einer Zustandsveränderung während oder nach einer definierten Scherbelastung. Beispielsweise kann eine Zustandsänderung, z.B. der Grad der Veränderung oder Schädigung als Funktion fortschreitender Scherbelastung bestimmt und dargestellt werden. Die Änderung von Meßwerten mit im Rahmen eines Scherzyklus fortschreitender Scherbelastung kann beispielsweise Rückschlüsse auf die Art einer Veränderung oder Schädigung des flüssigen Mediums erlauben. Handelt es sich bei dem scherbelasteten flüssigen Medium um ein flüssiges Überzugsmittel kann beispielsweise eine Beziehung zu der innerhalb einer Ringleitung herrschenden Scherbelastung hergestellt werden.

Die Untersuchungsergebnisse können beispielsweise zur Zustandscharakterisierung eines gegebenen flüssigen Mediums, beispielsweise eines flüssigen Überzugsmittels vor, während und nach einer definierten Scherbelastung, verwendet werden.

Das erfindungsgemäße Verfahren zur Ausübung einer Scherbelastung und zur anschließenden oder begleitenden Bestimmung der Scherbelastbarkeit eines flüssigen Überzugsmittels ist beispielsweise im Bereich der Pigment-, Lack- und Bindemittelentwicklung anwendbar. Es kann auch in der Qualitätskontrolle, beispielsweise bei der Chargenprüfüng in der Lackfertigung oder bei der Wareneingangskontrolle eingesetzt werden.

Das erfindungsgemäße Verfahren kann eingesetzt werden bei der Festlegung von Sollwerten für die Scherbelastbarkeit flüssiger Medien wie beispielsweise flüssiger Überzugsmittel.

Die Erfindung betrifft auch eine Vorrichtung zur Durchführung des erfindungsgemäßen Verfahrens in Form eines geschlossenen, das gegebene Volumen des flüssigen Mediums enthaltenden, entlüfteten Leitungssystems, das ein Scherelement für das flüssige Medium enthält. Dabei ist das Leitungssystem so gestaltet, daß die Scherbelastung des flüssigen Mediums bei Durchführung des erfindungsgemäßen Verfahrens im wesentlichen nur im Scherelement selbst erfolgt, d.h. die innere Oberfläche des Leitungssystems an sich einschließlich der inneren Oberfläche des Scherelementes ist glatt und ohne Grate. Bei dem Scherelement handelt es sich beispielsweise um eine Querschnittsverengung im Leitungssystem, beispielsweise in Form eines Spalts. Bevorzugt handelt es sich bei dem Scherelement um eines mit einem einstellbaren Querschnitt respektive einer einstellbaren Querschnittsverengung, beispielsweise ein mit einer Mikrometerschraube einstellbarer Spalt.

Eine Ausführungsform der erfindungsgemäßen Vorrichtung ist ein als geschlossener Kreislauf für das flüssige Medium ausgebildetes Leitungssystem, das eine Pumpe für das flüssige Medium und ein Scherelement enthält. Ein vollständiger Kreislauf des gesamten Volumens des flüssigen Mediums in dem so aufgebauten Leitungssystem entspricht einer Scherelementpassage.

Eine bevorzugte Ausführungsform der erfindungsgemäßen Vorrichtung ist ein Leitungssystem, das aus zwei über eine ein Scherelement für das flüssige Medium enthaltende Leitung miteinander verbundenen Zylindern mit Kolben besteht. Zweckmäßig handelt es sich um zwei Zylinder gleichen Rauminhalts. Die Zylinder können jeweils vollständig mittels eines Stempels, dessen Antrieb beispielsweise pneumatisch ist, entleert werden. Bei Start eines Scherzyklus ist das entlüftete Leitungssystem mit einem gegebenen Volumen des flüssigen Mediums befüllt, der Kolben des einen Zylinders ist vollständig eingeschoben, während der andere entsprechend der Füllmenge ausgeschoben ist. Das gegebene Volumen des flüssigen Mediums kann nun im Pendelverfahren unter wiederholtem Wechsel der Fließrichtung vom einen in den anderen Zylinder gedrückt werden. Zwecks jeweils möglichst vollständiger Scherelementpassage weist die die beiden Zylinder verbindende und das Scherelement enthaltende Verbindungsleitung einen Totraum mit nur geringem Rauminhalt auf, beispielsweise unter 5%, bevorzugt unter 1% des gegebenen Volumens an flüssigem Überzugsmittel. Der Totraum kann beispielsweise durch Wahl kurzer Verbindungsleitungen mit relativ kleinem, jedoch keine Scherung verursachenden Querschnitt niedrig gehalten werden.

Für inline-Druck- und inline-Temperaturmessungen können übliche Meßgeräte eingebaut sein. Für die Messung rheologischer Daten oder der Resonanzfrequenz des flüssigen Mediums kann insbesondere eine Meßeinheit mit Schwingungsgeber (Resonator), beispielsweise ein Piezoelement eingebaut sein, zweckmäßig in Baueinheit mit dem Scherelement.

Figur list eine schematische Querschnittsdarstellung eines Beispiels für die bevorzugte Ausführungsform der erfindungsgemäßen Vorrichtung, die zur Durchführung des erfindungsgemäßen Verfahrens geeignet ist. Diese besteht aus zwei über eine einen Querschnitt Q1 aufweisende Verbindungsleitung (1) verbundene Zylinder (2, 2') mit Kolben (5,5'). Die Verbindungsleitung enthält ein Scherelement (3) mit einstellbarem Querschnitt Q2 < Q1. Dargestellt ist außerdem ein Ventil (4) zur Probenentnahme. Die Vorrichtung ist entlüftet und mit einem flüssigen zu untersuchenden Medium (6), z.B. einem Lack gefüllt. Dessen Gesamtvolumen kann im Pendelverfahren unter wiederholtem Wechsel der Fließrichtung vom einen in den anderen Zylinder gedrückt werden.

Mit dem erfindungsgemäßen Verfahren und der erfindungsgemäßen Vorrichtung lassen sich flüssige Medien, insbesondere flüssige Überzugsmittel in definierter und reproduzierbarer Weise einer Scherbelastung aussetzen und reversible oder irreversible Veränderungen des flüssigen Mediums feststellen. Das erfindungsgemäße Verfahren ist reproduzierbar, es kann automatisiert durchgeführt werden. Je nach Ausführungsform des erfindungsgemäßen Verfahrens kann das scherbelastete flüssige Medium ohne oder nach Entnahme einer Probe auf die Auswirkung der Scherbelastung hin untersucht werden.

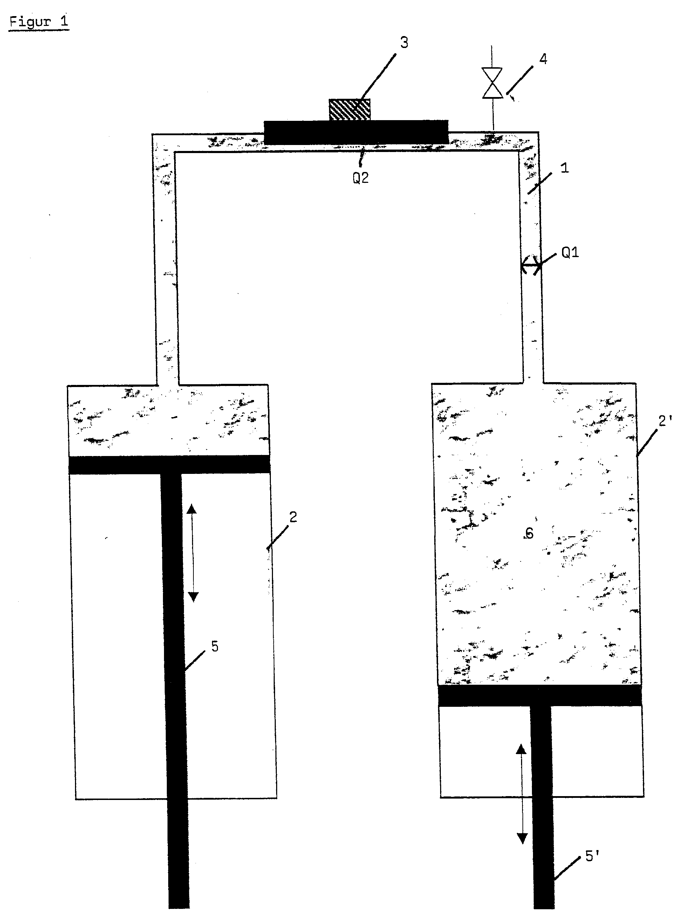

## Patentansprüche

1. Verfahren zur Feststellung von reversiblen oder irreversiblen Veränderungen eines flüssigen Mediums bei Scherbelastung, dadurch gekennzeichnet, daß man ein gegebenes Volumen des zu untersuchenden flüssigen Mediums wiederholt ein Scherelement unter reproduzierbaren Bedingungen passieren läßt.

2. Verfahren nach Anspruch 1, dadurch gekennzeichnet, daß man das flüssige Medium nach oder während der Scherbelastung auf reversible und/oder irreversible Veränderungen untersucht.

3. Verfahren nach Anspruch 1 oder 2, dadurch gekennzeichnet, daß der Anteil an pro Scherelementpassage nicht geschertem Volumen des flüssigen Mediums unter 5 % liegt.

4. Verfahren nach Anspruch 1,2 oder 3, dadurch gekennzeichnet, daß die Scherbelastung innerhalb eines geschlossenen, das gegebene Volumen des flüssigen Mediums enthaltenden entlüfteten Leitungssystems erfolgt und als Scherelement eine bestimmbare Querschnittsverengung innerhalb des geschlossenen Leitungssystems verwendet wird.

5. Verfahren nach einem der vorhergehenden Ansprüche, dadurch gekennzeichnet, daß man als flüssiges Medium ein Mehrphasensystem passieren läßt, das disperse flüssige und/oder feste Bestandteile in einer Flüssigkeit enthält.

6. Verfahren nach einem der Ansprüche 1 bis 5, dadurch gekennzeichnet, daß als flüssiges Medium ein flüssiges Überzugsmittel oder eine flüssige Komponente eines Überzugsmittels eingesetzt wird.

7. Verfahren nach einem der vorhergehenden Ansprüche, dadurch gekennzeichnet, daß es zur Untersuchung von reversiblen oder irreversiblen Schädigungen an flüssigen Überzugsmitteln oder deren flüssigen Komponenten durchgeführt wird, die durch Ringleitungen zum Sprühauftrag geführt werden sollen.

8. Vorrichtung zur Durchführung des Verfahrens nach einem der Ansprüche 1 bis 7 in Form eines geschlossenen, zur Aufnahme eines gegebenen Volumens eines flüssigen Mediums geeigneten, entlüftbaren Leitungssystems (1, 2, 2'), mit einem Scherelement (3) für flüssiges Medium und Einrichtungen zum wiederholten Passieren des gegebenen Volumens an flüssigem Medium durch das Scherelement (3).

9. Vorrichtung nach Anspruch 8 aus zwei über eine Verbindungsleitung (1) verbundenen Zylindern (2,2') mit Kolben (5,5'), wobei die Verbindungsleitung (1) ein Scherelement (3) enthält.

10. Vorrichtung nach Anspruch 8 oder 9, dadurch gekennzeichnet, daß sie eine Einrichtung (4) zur Entnahme von Proben eines in der Vorrichtung enthaltenen flüssigen Mediums aufweist.
